# EUROPÄISCHE PATENTSCHRIFT

(11) **EP 1 100 465 B1**
(45) Veröffentlichungstag und Bekanntmachung des Hinweises auf die Patenterteilung: **24.11.2004**
(21) Anmeldenummer: 99928996.0
(22) Anmeldetag: 22.07.1999
(51) Int. Cl.: A61K 9/12, C09K 3/30

(54) **MEDIZINISCHE AEROSOLFORMULIERUNGEN**
MEDICINAL AEROSOL FORMULATIONS
FORMULATIONS POUR AEROSOLS A USAGE MEDICAL

(30) Priorität: 24.07.1998 CH 156598
(43) Veröffentlichungstag der Anmeldung: 23.05.2001
(73) Patentinhaber: JAGO RESEARCH AG, CH-4132 Muttenz (CH)
(72) Erfinder: KELLER, Manfred, D-79189 Bad Krozingen (DE); HERZOG, Kurt, CH-4055 Basel (CH); MÜLLER-WALZ, Rudi, D-79650 Schopfheim (DE); KRAUS, Holger, CH-4462 Rickenbach (CH)
(74) Vertreter: Zimmermann, Hans, Dr.
(86) Internationale Anmeldenummer: PCT/CH1999/000337
(87) Internationale Veröffentlichungsnummer: WO 2000/006121

(56) Entgegenhaltungen:
- EP-A- 0 885 943
- WO-A-93/17665
- US-A- 4 397 836
- "Aerosol propellants comprising nitrous oxide and/or carbon dioxide" RES. DISCL. (1978), 170, 58 CODEN: RSDSBB, XP002090730

## Beschreibung

Die vorliegende Erfindung betrifft ein druckverflüssigtes Treibmittelgemisch auf der Basis von Hydrofluoralkanen, Hydrofluoralkan enthaltende Aerosolformulierungen sowie ein Verfahren zur Herstellung der Aerosolformulierungen.

Viele Gase, wie z.B. Kohlendioxid und Stickstoff, lassen sich zwar unter Druck verflüssigen, eignen sich aber nicht als Treibmittel für Dosieraerosole, weil der Binnendruck im Behältnis mit zunehmender Entleerung sehr stark abnimmt. Aus diesem Grunde können nur solche Treibgase für medizinische Dosieraerosole Verwendung finden, die sich bei Raumtemperatur verflüssigen lassen und nur allenfalls zu einer geringfügigen Abnahme des Binnendrucks im Behältnis führen, wenn der Inhalt sukzessive abgesprüht wird. Hierzu zählen die kurzkettigen Alkane, wie z.B. Propan, Butan und Isobutan, sowie die Fluorchlorkohlenwasserstoffe (FCKWs), wie z.B. Trichlorfluormethan (F11), Dichlordifluormethan (F12) und 1,2-Dichlor-1,1,2,2-tetrafluorethan (F114).

Aus WO-A-93/17665 ist zwar eine Methode zur Verabreichung physiologisch wirksamer Verbindungen bekannt, bei der aus einem überkritischen flüssigen Lösungsmittel und dem Wirkstoff eine überkritische flüssige Lösung gebildet und diese dann in den unterkritischen Bereich übergeführt wird. Als überkritisches Lösungmittel wurde Kohlendioxid verwendet, wobei ausgesagt wird, dass sich neben Kohlendioxid auch Distickstoffoxid, Fluorchlorkohlenwasserstoffe wie Dichlordifluormethan und Trichlorfluormethan, Xenon, Schwefelhexafluorid, Ethanol, Aceton, Propan, Wasser und Gemische davon eignen.

In Research Disclosure (1978) 170, 58, XP-002090730 wurde ferner erwähnt, dass einige Fluorkohlenwasserstoffund Fluorchlorkohlenwasserstoff-Treibmittel in Aerosolprodukten wie Haarsprays, Deodorants und Antitranspirationsmitteln als Co-Treibmittel zusammen mit Kohlendioxid oder Distickstoffmonoxid verwendet werden können. Die als Beispiele genannten 2,2-Dichlor-1,1,1-trifluorethan (F123), 1,2-Dichlor-1,1-difluorethan (F132b), 2-Chlor-1,1,1-trifluorethan (F133a), 1,1-Dichlor-1-fluorethan (F141b) und 1-Chlor-1,1-difluorethan (F142b) sind chlorierte und zudem wenig gebräuchliche Treibmittel. Ein Haarspray, in dem als Treibmittelgemisch Trifluormonochlorethan (F133a) zusammen mit Kohlendioxid und/oder Distickstoffmonoxid verwendet wird ist auch aus US-A-4 397 836 bekannt.

Aufgrund der Ozonproblematik, hervorgerufen durch die Abspaltung von radikalischen Chloratomen aus den FCKWs, haben sich im Montrealer Abkommen viele Staaten darauf verständigt, die FCKWs als Treibmittel zukünftig nicht mehr zu verwenden. Als FCKW-Ersatzstoffe für den medizinischen Bereich eignen sich fluorierte Alkane (im Rahmen der vorliegenden Erfindung auch als HFA bezeichnet), vor allem 1,1,1,2-Tetrafluorethan (HFA 134a) und 1,1,1,2,3,3,3-Heptafluorpropan (HFA 227), da diese inert sind und eine sehr geringe Toxizität aufweisen. Aufgrund ihrer physikalischen Eigenschaften, wie Druck, Dichte etc. sind sie besonders geeignet, um die FCKWs wie F11, F12 und F114 als Treibmittel in Dosieraerosolen zu ersetzen.

In der älteren EP-A1-0 885 943 wird ein Treibgasgemisch für Nahrungsmittel beschrieben, das aus einer mehrheitlichen Menge an 1,1,1,2-Tetrafluorethan und einer minderheitlichen Menge an Distickstoffmonoxid und/oder Kohlendioxid besteht.

In US-A-4 139 607 wurde andererseits ein Treibmittelsystem aus verflüssigtem Bis(difluormethyl)ether und gasförmigem Kohlendixoid vorgeschlagen, das im Gegensatz zu Kombinationen von Kohlendioxid mit anderen bekannten Treibmitteln wie Trichlorfluormethan oder Methylenchlorid befriedigende Aerosolmuster ergeben soll, das sich aber nicht durchgesetzt hat. Das Dokument erwähnt zwar, dass dem Treibmittelsystem andere Treibmittel wie Distickstoffmonoxid, Kohlenwasserstoffe und Fluorkohlenwasserstoffe oder flüssige Träger, wie Ethanol, Perchlorethylen, Trichlorethylen, Aceton, Amylacetat, Wasser und dergleichen, zugesetzt werden können; die offenbarten Formulierungen enthalten aber zumeist etwa 50% an Ethanol. Im Derwent-Abstract AN 89-184245 wird lediglich ausgesagt, dass in Aerosoldruckpackungen zur Verabreichung von Medikamenten anstelle von FCKWs auch Kohlenwasserstoffe, wie Butan und Pentan, andere Druckgase, wie Kohlendioxid, Dimethylether, Stickstoff und Distickstoffoxid, oder Fluorkohlenwasserstoffe verwendet werden könnten.

Medizinische Aerosolzubereitungen mit Hydrofluoralkanen wie HFA 134a sind bereits durch die Lehre von US-A-2 868 691 und US-A-3 014 844 umfasst und aus DE-A-2 736 500 und EP-A-0 372 777 bekannt. Beispiele von Formulierungen mit HFA 227 finden sich z.B. in WO-A-91/11495, EP-A-0 504 112 und EP-B-0 550 031. Es ist aus verschiedenen Veröffentlichungen bekannt, dass sich die üblichen, in FCKW-haltigen Dosieraerosolen verwendeten Hilfsstoffe, wie z.B. Lecithin, Sorbitantrioleat und Ölsäure, nur unzureichend in Hydrofluoralkanen wie z.B. HFA 134a und HFA 227 lösen, weil eine Kettenverlängerung und die Substitution der Chloratome durch Fluoratome zu einer Verschlechterung der Löslichkeitseigenschaften für die genannten zugelassenen Hilfsstoffe führt. Bereits bei den FCKWs, die im Vergleich zu den HFAs erheblich bessere Lösungsmittel darstellen, wurden zur Verbesserung der Löslichkeit oft Ethanol oder andere Cosolventien zugesetzt, um Arzneistoffe wie z.B. Isoprenalin und Epinephrin (vgl. US-A-2 868 691) als Aerosol applizieren zu können. Es war daher naheliegend, nicht nur die Löslichkeit der FCKWs, sondern auch diejenige der hFAs durch Zugabe von Ethanol zu verbessern. Beispiele hierfür finden sich in der Fachliteratur als auch in verschiedenen Patentanmeldungen. Alternativ dazu gibt es eine Reihe von Entwicklungen druckverflüssigter Aerosolzubereitungen mit HFA 134a und/oder HFA 227, die treibgaslösliche Hilfsstoffe, wie z.B. fluorierte oberflächenaktive Stoffe (WO-A-91/04011), mono- oder diacetylierte Glyceride (EP-A-0 504 112) oder polyethoxylierte Verbindungen (WO-A-92/00 061), verwenden, die sich auch ohne Ethanolzusatz in den beiden Treibgasen in der erforderlichen Menge lösen lassen.

Für FCKW-freie medizinische Aerosolzubereitungen mit einem hohen Dampfdruck wird heute als Treibgas meist HFA 134a (Dampfdruck ca. 6 bar bei 20°C) bevorzugt und für solche mit niedrigerem Dampfdruck HFA 227 (Dampfdruck ca. 4,2 bar bei 20°C). Beide Treibgase unterscheiden sich hinsichtlich ihrer Dichte (ca. 1,4 mg/ml für HFA 227 und ca. 1,2 mg/ml für HFA 134a bei 20°C), was insbesondere für Suspensionen von Bedeutung ist. Besitzt der Wirkstoff eine höhere Dichte als das Treibgas, kommt es zu einer Sedimentation; ist dessen Dichte geringer, kommt es zu einer Flotation. Zur Problemlösung bietet es sich deshalb an, unter Umständen Treibgas-Mischungen zu verwenden und/oder zur Erniedrigung der Dichte Cosolventien wie Ethanol, Diethylether oder andere niedrig siedende Lösungsmittel oder Treibgase wie z.B. n-Butan zuzusetzen. Ein wesentlicher Nachteil der Hydrofluoralkane ist deren geringeres Lösungsvermögen im Vergleich zu den FCKWs, insbesondere im Vergleich zu F11. Die Solvenzeigenschaften nehmen mit zunehmender Kettenlänge in der Reihenfolge F11 > HFA 134a > HFA 227 ab. Aus diesem Grunde lassen sich die üblicherweise in FCKWs verwendeten Suspendierhilfsmittel, wie Sorbitantrioleat, Lecithin und Ölsäure, ohne Erhöhung der Hydrophilie durch Zugabe von polaren Lösungsmitteln, wie z.B. Ethanol, nicht mehr in den üblichen Konzentrationen (Gewichtsverhältnisse von typischerweise etwa 1:2 bis 1:20, bezogen auf den Wirkstoff) lösen.

Es ist allgemein bekannt, dass im Falle von Suspensionsformulierungen nur Wirkstoffteilchen, die kleiner als 6 µm sind, lungengängig sind. Zur gewünschten Deposition derselben in der Lunge, müssen diese deshalb vor der Verarbeitung mittels spezieller Verfahren, wie z.B. mit Stift-, Kugel- oder Luftstrahlmühlen zerkleinert bzw. mikronisiert werden. Ein Mahlprozess führt in der Regel zu einer Oberflächenvergrösserung, die mit einer Erhöhung der elektrostatischen Ladung des mikronisierten Wirkstoffes einhergeht, wodurch meistens das Fliessverhalten und die Wirkstoff-Dispergierung verschlechtert wird. Als Folge der Grenz- und Ladungsaktivitäten kommt es häufig zu einer Agglomeration von Wirkstoffpartikeln oder auch zu Adsorption von Wirkstoff an Grenzflächen, die z.B. in der Anlagerung an Geräte oder Behälteroberflächen augenfällig wird.

In Aerosolzubereitungen, bei denen der Wirkstoff in verflüssigtem Treibgas suspendiert vorliegt, kann es zu einer Adsorption bzw. Ringbildung im Behälter an der Stelle kommen, wo die Flüssigphase in die Gasphase übergeht. Ohne Benetzung der mikronisierten Wirkstoffpartikel oder Abführen von Ladungen sowie Modifikation ihrer Oberflächeneigenschaften können Probleme bei der Dispergierung bzw. Suspendierung in den genannten Hydrofluoralkanen auftreten. Die mangelhafte Benetzung bzw. Dispergierung der Wirkstoffpartikel hat auch zur Folge, dass diese in vielen Fällen eine hohe Adsorptionstendenz aufweisen und an Oberflächen, wie z.B. der Behälterinnenwand oder dem Ventil kleben, was dann zu einer Unterdosierung sowie einer schlechten Dosiergenauigkeit von Sprühstoss zu Sprühstoss führt. Bei Suspensionen ist es deshalb in der Regel erforderlich, einen oberflächenaktiven Stoff oder ein Gleitmittel zuzusetzen, um die Adsorption an Grenzflächen zu erniedrigen, die Suspensionen zu stabilisieren und die Dosiergenauigkeit sicherzustellen. Besonders problematisch ist eine im Laufe der Lagerung eintretende Veränderung bzw. Erniedrigung des Anteils der inhalierbaren, lungengängigen Teilchen, der sogenannten Fine Particle Fraction (FPF) bzw. Fine Particle Dose (FPD), was zu einer Abnahme der Wirksamkeit der HFA-Zubereitung führt.

Zur Überwindung der oben dargelegten Probleme werden deshalb in der Regel oberflächenaktive Substanzen zugesetzt, wie sie bereits früher bei den FCKW-haltigen Formulierungen Anwendung fanden. Alternativ dazu kann in gewissen Fällen eine Modifikation der Oberflächeneigenschaften durch verschiedene Massnahmen (z.B. Coating) helfen, diese unerwünschten Effekte zu minimieren. Weil sich aber oberflächenaktive Mittel wie Ölsäure, Sorbitantrioleat und Lecithin nur unzureichend in Hydrofluoralkanen wie HFA 134a und HFA 227 lösen, wird bzw. muss in vielen Fällen Ethanol als Cosolvens zugesetzt werden, damit man die pharmazeutisch-technologischen Probleme besser kontrollieren kann.

Wird allerdings Ethanol in höherer Konzentration zugesetzt, erniedrigt sich die Dichte der Treibgas-Mischung, was vor allem bei Suspensionen zu einer unerwünschten Wirkstoff-Sedimentation führen kann. Zudem kann man unerwünschterweise einen "nassen Spray" erhalten, weil das Treibgas viel schneller verdampft als Ethanol. Daneben kann es aber durch die Erhöhung der Löslichkeit während der Lagerung auch zu Anlösungseffekten der Wirkstoffe kommen, was dann zu einem Kristallwachstum und damit wiederum zu einer Erniedrigung der Menge an inhalierbaren, lungengängigen Teilchen, der sogenannten Fine Particle Dose (FPD) führt.

Zur Messung der aerodynamischen Partikelgrössenverteilung bzw. des Anteils der in der Lunge deponierbaren Dosis, der sogenannten Fine Particle Dose (FPD), an inhalierbaren, lungengängigen Teilchen in einem Aerosol eignen sich Impaktoren, wie z.B. der 5-Stufen Multistage Liquid Impinger (MSLI) oder der 8-Stufen Andersen Kaskaden Impaktor (ACI), die in Chapter <601> der United States Pharmacopoeia (USP) oder in der Inhalanda Monographie der Europäischen Pharmacopoe (Ph. Eur.) beschrieben sind. Mit diesen Geräten lässt sich im Labor (in vitro) das aerodynamische Depositionsverhalten der Aerosolwolke untersuchen. Mittels eines "Log-probability plots" (logarithmische Darstellung der Wahrscheinlichkeitsverteilung) lässt sich dann der mittlere aerodynamische Teilchendurchmesser (Mass Median Aerodynamic Diameter MMAD) von Aerosol-Zubereitungen berechnen. Daraus kann man ableiten, ob der Wirkstoff eher im oberen oder unteren Lungenbereich deponiert wird.

Liegt der Wirkstoff im HFA-Treibgas/Ethanol-Gemisch nicht suspendiert, sondern gelöst vor, sind Probleme in Bezug auf die Streuung der Dosiergenauigkeit pro Hub meist weniger ausgeprägt. Wird hierzu jedoch eine grössere Menge Ethanol verwendet, kommt es beim Leerspülen des Behälters zu einem "Headspace"-Effekt wie folgt: Der Anteil on Ethanol, das einen geringeren Dampfdruck und eine geringere Dichte aufweist, nimmt zu und derjenige von Treibgas mit höherer Dichte und höherem Dampfdruck nimmt ab. Beim Absprühen bzw. mit zunehmender Behälterentleerung verändert sich das Konzentrationsverhältnis von Treibgas zu Ethanol, was aufgrund des Dichteunterschiedes zu einer Erniedrigung der Sprühstossmasse und damit auch des Sprühstoss- bzw. Wirkstoffgehaltes führt. Nachteilig ist des weiteren, dass bei höheren Ethanolkonzentrationen von z.B. 10%-30% der Anteil inhalierbarer Partikel (< 6 µm) meist abnimmt, weil der Spray aufgrund der andersgearteten Verdampfungseigenschaften von Ethanol im Vergleich zum Treibgas Tröpfchen mit grösserem aerodynamischem Durchmesser ergibt. als Folge davon kommt es zu einer Erniedrigung der für die Wirksamkeit entscheidenden Fine Particle Dose (FPD).

In einem Lösungsaerosol mit gleichem Ethanolgehalt erhält man üblicherweise mit HFA 134a im Vergleich zu HFA 227 eine höhere Fine Particle Fraction (FPF), d.h. einen grösseren Prozentsatz inhalierbarer Tröpfchen, was auf den höheren Druck von HFA 134a zurückzuführen ist. Prinzipiell gilt, je höher der Binnendruck in der Aerosoldose, desto feiner ist das Teilchenspektrum der Aerosolwolke. Lösungsaerosole mit geringem Ethanolanteil haben deshalb bei Verwendung von feinen Vernebelungsdüsen in der Regel einen kleineren MMAD (0,8-1,5 µm) als Suspensionsaerosole (2-4 µm). Dies hängt damit zusammen, dass bei Lösungsaerosolen Tröpfchen und bei Suspensionsaerosolen Partikel in Form einer Aerosolwolke erzeugt werden.

Für die topische Applikation von Wirkstoffen im Bereich der Bronchien und Bronchiolen, sind Partikelgrössen von ca. 2 - 4 µm vorteilhaft, wie sie üblicherweise mit Suspensionsformulierungen erreicht werden. Kleinere Partikel, die in den Alveolarbereich gelangen, werden zum Teil exhaliert (< 0,5 µm) oder gelangen durch Absorption in den systemischen Kreislauf. Hieraus folgt, dass Aerosolzubereitungen für die systemische Applikation günstigerweise Partikelgrössen von ca. 0,5 µm - 2 µm aufweisen sollten, wobei z.B. ein monodisperses Aerosol mit einem sehr hohen Anteil an Partikeln im Bereich von ca. 1 µm besonders vorteilhaft wäre. Abhängig vom gewünschten Depositionsort ist deshalb ein kleinerer oder grösserer MMAD sowie gegebenenfalls ein monodisperses Verteilungsspektrum bevorzugt. Hinsichtlich der Aerodynamik gilt: Je grösser die Masse der Partikel desto grösser ist ihre Tendenz geradlinig weiterzufliegen. Hieraus ergibt sich, dass es bei einer Änderung der Strömungsrichtung zur Impaktion von Teilchen kommt. Aus Depositionsstudien ist bekannt, dass selbst bei einem optimalen Inhalationsmanöver nur ca. 20% der aus einem Dosieraerosol emittierten Teilchen in die Lunge gelangen und nahezu 80 % im Oropharynx impaktieren.

Bei ethanolhaltigen Lösungsaerosolen kommt es leider häufig zu Problemen betreffend der Wirkstoffstabilität. Hiervon sind Wirkstoffe, wie z.B. Fenoterol und Salbutamol betroffen, weshalb solche Wirkstoffe bislang bevorzugt als Suspensionen formuliert wurden. Zur Reduktion ihrer Löslichkeit im Treibgasgemisch werden auch häufig die polareren Salze wie z.B. Fenoterol-Hydrobromid eingesetzt.

Der Erfindung liegt daher die Aufgabe zugrunde, ein Treibgassystem bereitzustellen, mit dem man:
- Wirkstoffe besser benetzen kann;
- Suspensionsaerosole mit verbesserten Suspensions- und Haltbarkeitseigenschaften herstellen kann;
- Lösungsaerosole mit verbesserter Lagerungsstabillität und geringerem Ethanolzusatz herstellen kann;
- die Dosiergenauigkeit verbessern kann;
- das Partikelgrössenverteilungsspektrum und den MMAD besser einstellen kann; und/oder
- die Fine Particle Dose (FPD) erhöhen und die oropharyngeale Deposition erniedrigen kann.

Diese Aufgabe wird gelöst durch ein druckverflüssigtes Treibmittelgemisch für Aerosole, umfassend Distickstoffmonoxid und ein Hydrofluoralkan der allgemeinen Formel

CₓH_{y}F_{z} (I)

worin x für die Zahl 1, 2 oder 3 steht, y und z je eine ganze Zahl ≥ 1 bedeuten und y + z = 2x + 2 ist.
Treibmittelgemische wie in Anspruch 1 definiert sind neu und Gegenstand der vorliegenden Erfindung.

Überraschenderweise wurde nämlich gefunden, dass man die genannten Ziele erreichen und Treibgasgemische mit vorteilhafteren Eigenschaften erhalten kann, wenn man Treibgasen auf der Basis von Hydrofluoralkanen eine geringe Menge an Distickstoffmonoxid (Lachgas) zusetzt. Gewünschtenfalls kann dem Treibmittel zusätzlich eine geringe Menge an Kohlendioxid zusetzt werden, das ähnliche Verbesserungen bewirkt. Derartige Gasgemische zeigen - im Unterschied zu Distickstoffoxid oder Kohlendioxid als alleiniges Treibgas - bei zunehmender Entleerung nur eine geringfügige Abnahme des Binnendrucks im Behältnis, was deren Verwendung als Treibmittel für Dosieraerosole ermöglicht. Wie in Tabelle 1 anhand einiger Beispiele veranschaulicht wird, lassen sich derartige Treibgasmischungen in einem breiten Temperaturbereich für Dosieraerosole einsetzen. Dieser Effekt wird auch beobachtet, wenn das Treibgasgemisch bzw. die Aerosolformulierung zusätzlich ein Cosolvens wie z.B. Ethanol enthält.

**Tabelle 1**

| Temperaturabhängigkeit N₂O-haltiger Hydrofluoralkane mit oder ohne Ethanol (EtOH) als Cosolvens | | | | | | | | |
|---|---|---|---|---|---|---|---|---|
| Gewichtsteile | | | | Druck (bar) bei | | | | |
| HFA227 | HFA134a | N₂O | EtOH | 9°C | 20°C | 30°C | 40°C | 50°C |
| 600 | 0 | 2 | 0 | 2,00 | 3,75 | 5,25 | 7,25 | 9,50 |
| 600 | 0 | 6 | 0 | 2,25 | 4,50 | 6,00 | 8,25 | 10,50 |
| 555 | 0 | 2 | 45 | 1,50 | 3,25 | 4,75 | 6,75 | 8,25 |
| 555 | 0 | 6 | 45 | 2,00 | 4,00 | 5,50 | 7,25 | 9,25 |
| 420 | 0 | 2 | 180 | 1,00 | 2,50 | 3,75 | 5,00 | 6,50 |
| 420 | 0 | 6 | 180 | 1,75 | 3,25 | 4,50 | 6,00 | 7,50 |
| 420 | 0 | 12. | 180 | 2,75 | 4,50 | 6,00 | 7,50 | 9,25 |
| 0 | 600 | 2 | 0 | 2,50 | 5,50 | 7,50 | 10,00 | 12,50 |
| 0 | 600 | 6 | 0 | 3,00 | 6,00 | 8,00 | 10,50 | 13,75 |
| 240 | 360 | 2 | 0 | 2,50 | 5,00 | 7,00 | 9,25 | 12,00 |
| 240 | 360 | 6 | 0 | 3,00 | 5,50 | 7,50 | 10,00 | 13,00 |
| 0 | 420 | 2 | 180 | 2,50 | 4,50 | 6,00 | 8,00 | 10,25 |
| 0 | 420 | 6 | 180 | 3,00 | 5,25 | 6,75 | 8,75 | 11,00 |

Überraschenderweise wurde ferner gefunden, dass durch die Zugabe von Distickstoffoxid und gewünschtenfalls Kohlendioxid zu Hydrofluoralkanen wie HFA 134a und/oder HFA 227 die Suspendierung von pharmazeutischen Wirkstoffen erleichtert und die Adhäsionstendenz und Adsorption von Wirkstoffen an Grenzflächen vermindert wird. Mit derartigen Treibgasgemischen lassen sich daher leichter Suspensionen herstellen, die sich durch eine kontrollierte Flockung auszeichnen, und als Folge der besseren Suspendiereigenschaften kann in vielen Fällen auf die Zugabe von - zum Teil unerwünschten - oberflächenaktiven Suspendierhilfsmitteln und/oder Cosolventien verzichtet oder zumindest deren Anteil verringert werden. Durch Zugabe von Gleitmitteln wie Glycerin oder Polyethylenglykol können oft Suspensions- oder Lösungsaerosole mit verbesserten Eigenschaften erhalten werden können.

Des weiteren wurde gefunden, dass die unerwünschte Deposition von Wirkstoff im Oropharynx reduziert und gleichzeitig die FPD erhöht werden kann.

Mit Hilfe von Distickstoffoxid und gewünschtenfalls Kohlendioxid ist es auch möglich, Sauerstoff aus den Hydrofluoralkanen zu verdrängen, wodurch die Lagerstabilität von oxidationsempfindlichen Wirkstoffen verbessert wird. Darüberhinaus kann man durch Zugabe von Distickstoffoxid und gewünschtenfalls Kohlendioxid den Binnendruck im Aerosolbehältnis so einstellen, dass man im Vergleich zu einem konventionellen FCKW- oder HFA-Dosieraerosol die FPF und den MMAD quasi so ausrichten kann, wie es für die jeweilige Anwendung am sinnvollsten erscheint. Es ist somit möglich, MDIs (Metered Dose Inhalers) sowohl für topische als auch systemische Applikationen herzustellen. Insbesondere für die systemische Applikation eröffnen sich völlig neue Anwendungsmöglichkeiten, weil man in Verbindung mit geeigneten Vernebelungsdüsen quasi monodisperse Aerosole mit hohen respirablen Fraktionen herstellen kann.

Das Treibmittelgemisch bietet somit auch bei Suspensions- und Lösungsaerosolformulierungen Vorteile, bei denen ein oberflächenaktives Mittel und/oder ein Cosolvens nötig oder erwünscht ist. Einerseits gestattet die Verwendung von Treibmitteln, die Distickstoffoxid und gewünschtenfalls Kohlendioxid enthalten, häufig eine Verringerung der benötigten Cosolvensmenge und eine bessere Löslichkeit herkömmlicher oberflächenaktiver Mittel. Anderseits kann der nachteilige Einfluss von Cosolventien wie Ethanol auf die Tröpfchengrösse ganz oder weitestgehend vermieden werden, da durch eine entsprechende Erhöhung der Konzentration an Distickstoffoxid und gewünschtenfalls Kohlendioxid auch bei vergleichsweise hohen Cosolvenskonzentrationen der Binnendruck und das Depositionsverhalten so eingestellt werden kann, dass sowohl die Fine Particle Dose als auch der MMAD therapiegerecht eingestellt werden können.

Die Herstellung der Treibmittelgemische kann in an sich bekannter Weise dadurch erfolgen, dass man Distickstoffmonoxid und gewünschtenfalls Kohlendioxid unter Druck in ein Hydrofluoralkan der Formel I einleitet.

Das Treibmittelgemisch eignet sich grundsätzlich für beliebige Aerosolanwendungen wie beispielsweise kosmetische und Haushaltssprays. Aufgrund der beschriebenen Vorteile - wie geringer Abfall des Binnendruckes bei Entleerung, geringere Temperaturabhängigkeit und leichtere Einstellbarkeit des Binnendruckes, verbesserte Benetzungseigenschaften für pharmazeutische Wirkstoffe und Verwendbarkeit herkömmlicher oberflächenaktiver Mittel wie Ölsäure, Lecithin und Sorbitantrioleat - ist das Treibmittelgemisch aber vor allem auch für medizinische Aerosolformulierungen und insbesondere für Inhalationsaerosole geeignet.

Die Erfindung betrifft daher ebenfalls eine medizinische Aerosolformulierung, umfassend eine wirksame Menge eines pharmazeutischen Wirkstoffes und ein druckverflüssigtes Treibmittelgemisch, enthaltend Distickstoffmonoxid und ein Hydrofluoralkan der allgemeinen Formel

CₓH_{y}F_{z} (I)

worin x für die Zahl 1, 2 oder 3 steht, y und z je
eine ganze Zahl ≥ 1 bedeuten und y + z = 2x + 2 ist.

Beispiele geeigneter Hydrofluoralkane, die in den erfindungsgemässen Treibmittelgemischen und Aersolformulierungen verwendet werden können, sind: Difluormethan (HFA 32), Pentafluorethan (HFA 125), 1,1,2,2-Tetrafluorethan (HFA 134), 1,1,1,2-Tetrafluorethan (HFA 134a), 1,1,2-Trifluorethan (HFA 143), 1,1,1-Trifluorethan (HFA 143a), 1,1-Difluorethan (HFA 152a), 1,1,1,2,3,3,3-Heptafluorpropan (HFA 227), Hexafluorpropan (HFA 236), Pentafluorpropan (HFA 245) und dergleichen. Im allgemeinen sind Hydrofluoralkane mit 2 oder 3 Kohlenstoffatome bevorzugt. Besonders bevorzugt sind Treibmittelgemische und Aerosolformulierungen, die 1,1,1,2-Tetrafluorethan (HFA 134a), 1,1,1,2,3,3,3-Heptafluorpropan (HFA 227) oder ein Gemische der beiden, beispielsweise ein 1:1-Gemisch, enthalten.

Die erfindungsgemässen Treibmittelgemische und Aerosolformulierungen enthalten vorzugsweise mindestens etwa 0,0001 Gew.-%, insbesondere mindestens etwa 0,01 Gew.-% an Distickstoffmonoxid. Gewünschtenfalls können die Treibmittelgemische und Aerosolformulierungen zusätzlich eine geringe Menge an Kohlendioxid enthalten. Der Gehalt an Distickstoffmonoxid und Kohlendioxid ist u.a. abhängig vom gewünschten Druck, der Art der verwendeten Hydrofluoralkane der Art und Menge allfälliger weiterer Treibgase und Cosolventien und dergleichen. Im allgemeinen beträgt jedoch der Gehalt an Distickstoffmonoxid bzw. der Gehalt and Distickstoffmonoxid und Kohlendioxid zusammen etwa 0,0001 bis 10 Gew.-%, vorzugsweise etwa 0,01 bis 6 Gew.-% und besonders bevorzugt etwa 0,1 bis 3 Gew.-%. Im Falle von medizinischen Aerosolen und insbesondere bei Inhalationsaerosolen ist im allgemeinen ein Gehalt an Distickstoffmonoxid bzw. an Distickstoffmonoxid und Kohlendioxid zusammen von etwa 0,01 bis 2 Gew.-%, typischerweise etwa 0,1 bis 1,0 Gew.-%, bevorzugt; höhere Konzentrationen sind in der Regel nur dann angezeigt, wenn die Formulierung einen vergleichsweise hohen Anteil an Cosolventien wie Ethanol oder Wasser enthält.

Der Ausdruck "pharmazeutische Wirkstoff" umfasst im Rahmen der vorliegenden Erfindung therapeutische Wirkstoffe als auch Impfstoffe und andere Stoffe zur Gesundheitsprophylaxe. Als pharmazeutische Wirkstoffe für die erfindungsgemässen Aerosolformulierungen eignen sich grundsätzlich alle als Aerosol verabreichbaren Wirkstoffe, wie Betamimetika, Corticosteroide, Anticholinergika, Cyclooxigenase-, Mastzell-, Lipoxigenase- und Proteolytische Enzym-Inhibitoren, Arachidonsäure-, Leukotrien-, Thromboxan-, Natrium/Kaliumkanal-, Neurokinin-, Tachykinin-, Bradykinin-, Muscarin,- Histamin-, Phosphodiesterase-, Platelet-Activating-Faktor- und Selectin-Antagonisten, Kaliumkanalblocker, Antiinfektiva, Antibiotika, Pentamidin, Cytostatika, Fungistatika, Radikalfänger, Vitamine, Hormone, Immunstimulantien, Immunsuppresiva, Mucolytika, Heparin, Antidiabetika, Analgetika, Schlafmittel und dergleichen, beispielsweise
- Betamimetika wie Salbutamol, Formoterol, Salmeterol, Fenoterol, Clenbuterol, Terbutalin, Bambuterol, Broxaterol, Epinephrin, Isoprenalin, Orciprenalin, Hexoprenalin, Tolbuterol, Reproterol, Bamethan, Tetroquinol, Levalbuterol etc.,
- Corticoide wie Beclomethason, Dexamethason, Ciclomethason, Triamcinolon, Budesonid, Butixocort, Ciclesonid, Fluticason, Flunisolid, Icomethason, Mometason etc.,
- Anticholinergika und Spasmolytika wie Atropin, Glycopyrroniumbromid, Scopolamin, N-Butylscopolamin, Trospiumchlorid, Ipratropiumbromid, Oxitropiumbromid, Tiotropiumbromid, Droferin, Oxybutinin, Moxaverin etc.,
- Mastzell- und Histamininhibitoren wie Cromoglycinsäure, Nedocromil, Pemirolast etc. und 5-Lipoxigenasehemmer wie Zileuton, Linazolast etc.,
- Leukotrienantagonisten wie Iralukast, Zafirlukast, Montelukast, Roflumilast, Imitrodast, Ontozolast und Pranlukast, Natriumkanalantagonisten wie Amilorid, Kaliumkanalantagonisten wie Bimakalim, Arachidonsäureantagonisten wie 2-Benzoxazolamin, Histaminrezeptorantagonisten wie Epinastin, Cetrizin, Mizolastin und Mequitamium,
- Migränemittel wie Mutterkornalkaloide, Methysergid, Ergotamin, Serotonin, Sumatriptan, Zolmitriptan, Cyclandelat etc.,
- Analgetika wie Fentanyl, Morphin, Buprenorphin, Opium, Heroin, Nalbuphin, Pentazocin, Oxycodon, Tramadol, Pethidin, Tilidin, Methadon, Nefopam, Dextropropoxyphen, Piritramid etc.,
- Mucolytica wie R-nase, Acetylcystein, Ambroxol, Apafant, Bromhexin, Human Lung Surfactant etc.,
- Antiemetika wie Bromoprid, Domperidon, Metoclopramid, Triethylperazin, Trifluorpromazin, Meclozin, Chlorphenoxamin, Dimenhydrinat etc.,
- Antibiotika wie Penicilline (z.B. Azlocillin), Cephalosporine (z.B. Cefotiam oder Ceftriaxon), Carbapeneme, Monobatame, Aminoglykoside (z.B. Streptomycin, Neomycin, Gentamycin, Amikacin oder Tobramycin), Chinolone (z.B. Ciprofloxacin), Makrolide (z.B. Erytromycin), Nitroimidazole (z.B. Tinidazol), Lincosamide (z.B. Clindamycin), Glykopeptide (z.B. Vancomycin), Polypeptide (z.B. Bacitracin) etc.,
- Vitamine und Radikalfänger wie Vitamin A, B, C, D oder E, Katalase, Superoxidbismutase, reduziertes Glutathion etc.,
- Antidiabetika wie Gibenclamid, Glipizid, Gliclacid, Glimepirid, Troglitazone etc.,
- Schlafmittel wie Benzodiazepine, Piperidindione, Antihistaminika etc.,
- Neuroleptika, Antidepressiva und Antikonvulsiva wie Benzodiazepine, Phenothiazine, Butyrophenone, Sulpirid, Hydantoine, Barbiturate, Succinimide, Carbamazepin etc.,
- Hormone wie Androgene (z.B. Testosteron), Antioestrogene, Oestrogene (z.B. Estradiol), Gestagene (z.B. Progesteron), Corticosteroide, Calcitonin, Parathyrin, Somatotropin, Oxytocin, Prolactin, Glucagon, Erythropoietin, Atriopeptin, Melanotropin, Throtropin, Gonadotropin, Vasopressin, Insulin etc.,
- Potenzmittel wie Phentolamin, Sildenafil, Alprostadil etc.,
- Cytostatika wie Stickstofflostderivate (z.B. Ifosphamid), N-Nitrosoharnstoffderivate (z.B. Lomustin), Antagonisten von Purin-und Pyrimidinbasen (z.B. Fluorouracil), Platinkomplexe (z.B. Carboplatin), Anthracycline (z.B. Doxorubicin), Podophyllinderivate (Podophyllotoxin).

Die genannten Wirkstoffe können gegebenenfalls in Form ihrer Isomere, Enantiomere oder Racemate und im Falle von Säuren oder Basen als solche oder in Form ihrer pharmazeutisch annehmbaren Salze oder Derivate verwendet werden. Die optimale Wirkstoffmenge in den erfindungsgemässen Formulierungen hängt vom jeweiligen Wirkstoff ab. In der Regel sind jedoch Aerosolformulierungen bevorzugt, die mindestens etwa 0,0001 und höchstens etwa 5 Gew.-%, insbesondere etwa 0,01 bis 3 Gew.-%, an Wirkstoff enthalten.

Beispiele bevorzugt verwendbarer Wirkstoffe sind die Antiasthmatika wie z.B. Betamimetika, Corticosteroide und Anticholinergika und Antiallergika wie z.B. Mastzellinhibitoren. Besonders bevorzugt sind Aerosolformulierungen, die Salbutamol, Formoterol, Salmeterol, Fluticason, Budesonid, Ciclesonid, Glycopyrronium, Tiotropium, Cromoglycinsäure, Nedocromil, Mometason, Sildenafil, Beclomethason, Levalbuterol oder ein pharmazeutisch annehmbares Salz oder Derivat dieser Wirkstoffe enthalten.

Die erfindungsgemässen Aerosolformulierungen können je nach Art der Wirkstoffe und weiteren Zusätze in Form von Suspensionen, Emulsionen oder Lösungen vorliegen. Die Herstellung der Aerosolformulierungen kann in an sich bekannter Weise dadurch erfolgen, dass man Distickstoffmonoxid unter Druck in ein verflüssigtes Hydrofluoralkan der Formel I einleitet und den pharmazeutischen Wirkstoff zusetzt. Die Zugabe des Distickstoffmonoxids und des Wirkstoffes kann grundsätzlich in beliebiger Reihenfolge erfolgen. Im Falle von Suspensionsformulierungen ist es jedoch in der Regel bevorzugt, zuerst das Distickstoffmonoxid in das Treibmittel einzuleiten und dann den mikronisierten Wirkstoff zuzusetzen. Die Mikronisierung des Wirkstoffes kann in bekannter Weise erfolgen und wird vorzugsweise so durchgeführt, dass eine Partikelgrösse von etwa 0,5 bis 6 µm erhalten wird. Wird der Aerosolformulierung zusätzlich Kohlendioxid zugesetzt, so kann dieses unter Druck entweder separat oder zusammen mit dem Distickstoffmonoxid in das verflüssigte Hydrofluoralkan eingeleitet werden.

Die erfindungsgemässen Treibmittelgemische und Aerosolformulierungen können ein oder mehrere Hydrofluoralkane und gewünschtenfalls weitere Treibgase enthalten. Vorzugsweise enthalten sie jedoch keine Fluorchlorkohlenwasserstoffe. Besonders bevorzugt sind im allgemeinen solche Treibmittelgemische und Aerosolformulierungen, die - abgesehen von gewünschtenfalls als Cosolventien verwendbaren Verbindungen wie Wasser, niedere Alkane, niedere Alkohole und niedere Ether - als Treibgase lediglich Distickstoffmonoxid und ein oder mehrere Hydrofluoralkane der Formel I und gewünschtenfalls Kohlendioxid enthalten. Das Hydrofluoralkan bzw. die Hydrofluoralkane und die Kohlendioxidkonzentration werden vorzugsweise so gewählt, dass im Aerosolbehältnis ein Binnendruck von etwa 3 bis 10 bar, besonders bevorzugt etwa 3,5 bis 6 bar, bei 20°C eingestellt werden kann.

Die erfindungsgemässen Aerosolformulierungen eignen sich für Suspensions-, Emulsions- und Lösungsformulierungen, und sie können übliche Zusätze wie Cosolventien, Gleit- oder Schmiermittel (z.B. Glycerin) und oberflächenaktive Mittel enthalten. Die Zugabe des Wirkstoffes und allfälliger weiterer Zusätze kann in an sich bekannter Weise erfolgen. Infolge der erfindungsgemäss erzielbaren Verbesserung der Fine Particle Fraction und der gleichzeitigen Reduktion der unerwünschten oropharyngealen Deposition ist es häufig möglich, die Wirkstoffkonzentration im Vergleich zu einem FCKW-haltigen Dosieraerosol signifikant zu verringern.

Die Verwendung eines Cosolvens ist insbesondere bei Lösungsformulierungen häufig angezeigt, kann aber gelegentlich auch bei Suspensionsformulierungen von Vorteil sein. Als Cosolventien eignen sich insbesondere Wasser, niedere Alkohole, niedere Alkane und niedere Ether, vorzugsweise Wasser, Alkohole mit 1 bis 3 Kohlenstoffatomen, Alkane mit 3 bis 6 Kohlenstoffatomen und Dialkylether mit 2 bis 4 Kohlenstoffatomen, wie Wasser, Ethanol, Propanol, Isopropanol, Ethylenglykol, Propylenglykol, Glycerin, Propan, Butan, Isobutan, Pentan, Dimethylether, Diethylether und dergleichen. Besonders bevozugt sind Diethylether und insbesondere Ethanol. Der Anteil an Cosolvens in den erfindungsgemässen Treibmittelgemischen und Aerosolformulierungen kann, falls vorhanden, im allgemeinen etwa 0,01 bis 40 Gew.-%, insbesondere etwa 0,1 bis 15 Gew.-%, bezogen auf das Gesamtgemisch bzw. die Gesamtformulierung betragen.

Der Anteil an einem oder mehreren Hydrofluoralkanen der Formel I in den erfindungsgemässen Treibmittelgemischen und Aerosolformulierungen beträgt im allgemeinen mindestens etwa 40 Gew.-%, vorzugsweise mindestens etwa 64 Gew.-% und besonders bevorzugt mindestens etwa 87 Gew.-% des Gesamtgemisches bzw. der Gesamtformulierung. Im Falle der medizinischen Aerosolformulierungen kann jedoch der Anteil an Hydrofluoralkanen im Hinblick auf den Gehalt an Wirkstoff, oberflächenaktivem Mittel und allfälligen weiteren Zusätzen auch niedriger sein und beispielsweise mindestens etwa 30 Gew.-% betragen.

Die Verwendung eines oberflächenaktiven Mittels ist insbesondere bei Suspensionsformulierungen häufig angezeigt, kann aber auch bei Lösungsformulierungen z.B. zur Ventilschmierung von Vorteil sein. Grundsätzlich eignen sich alle gebräuchlichen oberflächenaktiven Mittel wie Ölsäure, Lecithin, Sorbitantrioleat, Cetylpyridiniumchlorid, Benzalkoniumchlorid, Polyoxyethylen(20)sorbitanmonolaurat, Polyoxethylen(10)stearylether, Polyoxyethylen(2)oleylether, Polyoxyethylen(20)sorbitanmonostearat, Polyoxyethylen(20)sorbitanmonooleat, Polyoxypropylen-polyoxyethylen-Blockcopolymere, Polyoxypropylen-polyoxyethylen-ethylendiamin-Blockcopolymere, ethoxyliertes Ricinusöl und dergleichen. Bevorzugt sind im allgemeinen Ölsäure, Sorbitantrioleat und Lecithin. Der Anteil an oberflächenaktivem Mittel kann, falls vorhanden, vorzugsweise etwa 0,0001 bis 1 Gew.-%, insbesondere etwa 0,001 bis 0,1 Gew.-%, bezogen auf die Gesamtformulierung, betragen. Vorzugsweise können aber die erfindungsgemässen Aerosolformulierungen auch im wesentlichen frei von oberflächenaktiven Mitteln sein, d.h. weniger als 0,0001 Gew.-% an oberflächenaktiven Mitteln enthalten.

Weiterhin können die erfindungsgemässen Aerosolformulierungen gewünschtenfalls Puffersubstanzen oder Stabilisatoren wie Citronensäure, Ascorbinsäure, Natrium-EDTA, Vitamin E, N-Acetylcystein und dergleichen enthalten. Im allgemeinen werden solche Substanzen, falls vorhanden, in Mengen von nicht mehr als etwa 1 Gew.-%, beispielsweise etwa 0,0001 bis 1 Gew.-%, bezogen auf die Gesamtformulierung, verwendet.

Die erfindungsgemässen Aerosolformülierungen können in an sich bekannter Weise unter Verwendung von Rührern und Homogenisatoren hergestellt werden. Zur Abfüllung können bekannte Verfahren, wie die Kalt- oder Druckfülltechnik oder Modifikationen dieser Techniken, eingesetzt werden. Als Behältnisse eignen sich beispielsweise drucksichere Behälter aus Glas, Kunststoff oder Aluminium, die mit Dosierventilen von z.B. 10 bis 140 µl bestückt und mit handelsüblichen - auch atemzuggetriggerten - Mundrohradaptern versehen werden können.

Die erfindungsgemäss verwendeten Treibmittelgemische bieten somit bei der Herstellung von Aerosolformulierungen eine Reihe von vorteilen, wie bessere Wirkstoffbenetzung, verbesserte Suspensions- und Haltbarkeitseigenschaften von Suspensionsformulierungen, Verbesserung der Dosiergenauigkeit, Erhöhung der Fine Particle Dose sowie gewünschtenfalls eine Verringerung der Cosolvensmengen bzw. die weitgehende Vermeidung der Nachteile hoher Cosolvensmengen.

Die Erfindung betrifft daher ebenfalls die Verwendung der Treibmittelgemische als Treibmittel für Aerosole, wie in den Ansprüchen 26-28 definiert. Nasale oder inhalative Aerosole können vorzugsweise einen aerodynamischen Partikel- bzw. Tröpfchendurchmesser von etwa 0,5 bis 40 µm, insbesondere etwa 0,5 bis 6 µm, aufweisen.

Mit dem erfindungsgemäss verwendeten Treibgas-System lässt sich beispielsweise ein Budesonid-Dosieraerosol herstellen, das im Vergleich zu einem FCKW-haltigen Handelsprodukt (Pulmicort®, Astra, Schweden) eine weitaus bessere Dosiergenauigkeit und eine fast doppelt so hohe FPF aufweist. Ergänzend dazu wird die Deposition im Mundrohr etwa halbiert und diejenige im "sample induction port" (artifizieller Oropharynx) von ca. 50% auf 20% reduziert. Die erfindungsgemässe Formulierung ermöglicht also das Dosieraerosol in Bezug auf mehrere Aspekte vorteilhafter zu formulieren, da die respirable Dosis quasi verdoppelt und die unerwünschte oropharyngeale in-vitro Deposition im "sample induction port" erniedrigt werden kann, wie es am Beispiel für Beclomethasondipropionat, Budesonid und Dinatriumcromoglycat gezeigt werden kann. Es ist daher zu erwarten, dass man im Falle von Budesonid vermutlich mit der halben Dosierung denselben therapeutischen Effekt erreicht, wie z.B. mit dem Handelsprodukt Pulmicort® .

Die Erfindung wird durch die folgenden Beispiele weiter veranschaulicht. Die Homogenisierung von Wirkstoffsuspensionen erfolgte jeweils mit einem Rotor-Stator-Homogenisator (Kinematika).

### Beispiel 1

100 g mikronisiertes Dinatriumcromoglykat werden in einen Druckansatzkessel eingewogen. Nach Verschliessen und Evakuieren des Ansatzkessels werden unter Rühren 8,5 kg HFA 227 zugegeben, das zuvor in einem anderen Druckansatzkessel mit 3 Gew.-% Ethanol versetzt und mit Distickstoffoxid begast und auf einen Druck von 5 bar (20°C) eingestellt wurde. Nach Homogenisieren wird die erhaltene Suspension mittels Druckfülltechnik in mit Dosier-Ventilen verschlossene Aluminiumdosen abgefüllt.

### Beispiel 2

2 g mikronisiertes Ipratropiumbromid werden in einen Druckansatzkessel eingewogen. Nach Verschliessen und Evakuieren desselben werden 6,0 kg eines Gemisches aus HFA 227 und HFA 134a (Gewichtsverhältnis 80:20) zugegeben, das zuvor in einem anderen Druckansatzkessel mit Distickstoffoxid begast und auf einen Druck von 5,5 bar bei 20°C eingestellt wurde. Nach Homogenisieren dieser Mischung wird die erhaltene Suspension mittels Druckfülltechnik in Behälter abgefüllt, die mit einem Dosierventil bestückt werden.

### Beispiel 3

5 g mikronisiertes Glycopyrroniumbromid werden in einen Druckansatzkessel eingewogen. Nach Verschliessen und Evakuieren desselben werden 10 kg HFA 227 zugegeben, das zuvor in einem anderen Druckansatzkessel mit 1 Gew.-% Ethanol versetzt und mit Distickstoffoxid begast und auf einen Druck von 5,25 bar (20°C) eingestellt wurde. Nach Homogenisieren dieser Mischung wird die erhaltene Suspension mittels Druckfülltechnik in mit Dosier-Ventilen verschlossene drucksichere Glasbehälter abgefüllt.

### Beispiel 4

0,6 g mikronisiertes Formoterol-fumarat und 20 g mikronisiertes Glycopyrroniumbromid werden in einen Druckansatzkessel eingewogen. Nach Verschliessen und Evakuieren des Ansatzkessels werden 6,5 kg eines Treibgasgemisches aus HFA 227 und HFA 134a (Gewichtsverhältnis 70:30), das zuvor in einem anderen Druckansatzkessel mit 2 Gew.-% Ethanol versetzt und mit Distickstoffoxid begast und auf einen Druck von 5,5 bar (20°C) eingestellt wurde, unter Rühren zugegeben. Nach Homogenisieren wird die erhaltene Suspension mittels Druckfülltechnik in mit Dosier-Ventilen verschlossene Aluminiumdosen abgefüllt.

### Beispiel 5

120 g Beclomethason-dipropionat werden in einen Ansatzkessel eingewogen und in 6 kg Ethanol gelöst, in dem zuvor 10 g Ölsäure gelöst wurden. Jeweils 1 g dieser Lösung wird in Aluminiumdosen abgefüllt und nachfolgend mit Dosierventilen verschlossen. In einem Druckansatzkessel wird HFA 227 mit Distickstoffoxid begast und auf einen Druck von 5,5 bar bei 20°C eingestellt. Von dieser Mischung werden jeweils 11 g pro Dose zugedrückt und diese danach im Ultraschallbad behandelt.

### Beispiel 6

10 g mikronisiertes Levalbuterol-sulfat werden in einen Druckansatzkessel eingewogen. Nach Verschliessen und Evakuieren desselben werden 13 kg HFA 227 zugegeben, das zuvor in einem anderen Druckansatzkessel mit 650 g Ethanol versetzt und mit Distickstoffoxid begast und auf einen Druck von 5,25 bar (20°C) eingestellt wurde. Nach Homogenisieren dieser Mischung wird die erhaltene Suspension in drucksichere Behälter abgefüllt, die mit Dosier-Ventilen bestückt werden.

### Beispiel 7

120 g Fluticason werden in einen Ansatzkessel eingewogen und in 6 kg Ethanol gelöst, in dem zuvor 6 g Ölsäure gelöst wurden. Jeweils 1,2 g dieser Lösung wird in drucksichere Behälter abgefüllt und nachfolgend mit Dosierventilen verschlossen. In einem Druckansatzkessel wird HFA 134a mit Distickstoffoxid begast und auf einen Druck von 5,5 bar bei 20°C eingestellt. Von dieser Mischung werden jeweils 12 g pro Dose zugedrückt und diese danach im Ultraschallbad behandelt.

### Beispiel 8

3,0 g mikronisiertes Budesonid werden in einen Druckansatzkessel eingewogen. Nach Verschliessen und Evakuieren desselben wird eine Mischung aus 0,85 kg HFA 134a und 0,85 kg HFA 227 zugegeben, die zuvor in einem anderen Druckansatzkessel mit Distickstoffoxid begast und auf einen Druck von 5,5 bar (20°C) eingestellt wurde. Nach Homogenisieren dieser Mischung wird die erhaltene Suspension mittels Druckfülltechnik in mit Dosier-Ventilen verschlossene Aluminiumdosen abgefüllt.

### Beispiel 9

3,0 g mikronisiertes Fluticason-propionat und 0,15 g mikronisiertes Formoterol-fumarat werden in einen Druckansatzkessel eingewogen. Nach Verschliessen und Evakuieren desselben wird eine Mischung aus 0,5 kg HFA 134a und 1,5 kg HFA 227 zugegeben, die zuvor in einem anderen Druckansatzkessel mit 2 Gew.-% Ethanol versetzt und mit Distickstoffoxid begast und auf einen Druck von 5,5 bar (20°C) eingestellt wurde. Nach Homogenisieren dieser Mischung wird die erhaltenen Suspension in drucksichere Behälter abgefüllt, die mit Dosier-Ventilen verschlossen werden.

### Beispiel 10

5 g mikronisiertes Salmeterol-Xinafoat und 2 g mikronisiertes Glycopyrroniumbromid werden in einen Druckansatzkessel eingewogen. Nach Verschliessen und Evakuieren des Ansatzkessels werden 70 kg HFA 227, das zuvor in einem anderen Druckansatzkessel mit 2 Gew.-% Ethanol versetzt und mit Distickstoffoxid begast und auf einen Druck von 5,5 bar (20°C) eingestellt wurde, unter Rühren zugegeben. Nach Homogenisieren wird die erhaltene Suspension mittels Druckfülltechnik in mit Dosier-Ventilen verschlossene Aluminiumdosen abgefüllt.

### Beispiel 11

10 g Sildenafil und 0,1 g δ-Tocopherol werden in einen Ansatzkessel eingewogen und in 100 g Ethanol gelöst, in dem zuvor 0,1 g Lecithin gelöst wurden. Jeweils 1 g dieser Lösung wird in drucksichere Behälter abgefüllt und nachfolgend mit Dosierventilen verschlossen. In einem Druckansatzkessel wird HFA 134a mit Distickstoffoxid begast und auf einen Druck von 6,5 bar bei 20°C eingestellt. Von dieser Mischung werden jeweils 7 g pro Dose zugedrückt, die danach im Ultraschallbad behandelt werden.

### Beispiel 12

120 g Beclomethason-dipropionat werden in einen Ansatzkessel eingewogen und in 6 kg Ethanol gelöst, in dem zuvor 120 g Glycerin gelöst wurden. Jeweils 1 g dieser Lösung wird in Aluminiumdosen abgefüllt und nachfolgend mit Dosierventilen verschlossen. In einem Druckansatzkessel wird HFA 227 mit Distickstoffoxid begast und auf einen Druck von 5,5 bar bei 20°C eingestellt. Von dieser Mischung werden jeweils 11 g pro Dose zugedrückt und diese danach im Ultraschallbad behandelt.

### Beispiel 13

10 g Sildenafil und 0,1 g δ-Tocopherol werden in einen Ansatzkessel eingewogen und in 100 g Ethanol gelöst, in dem zuvor 1 g Glycerin gelöst wurden. Jeweils 1 g dieser Lösung wird in drucksichere Behälter abgefüllt und nachfolgend mit Dosierventilen verschlossen. In einem Druckansatzkessel wird HFA 227 mit Distickstoffoxid begast und auf einen Druck von 6 bar bei 20°C eingestellt. Von dieser Mischung werden jeweils 6 g pro Dose zugedrückt, die danach im Ultraschallbad behandelt werden.

### Beispiel 14

1,6 g mikronisiertes Budesonid werden in einen Druckansatzkessel eingewogen. Nach Verschliessen und Evakuieren desselben wird eine Mischung aus 20 g Propylenglykol, 30 g Ethanol und 950 g HFA 227 zugegeben, die zuvor in einem anderen Druckansatzkessel mit Distickstoffoxid begast und auf einen Druck von 5,5 bar (20°C) eingestellt wurde. Nach Homogenisieren dieser Mischung wird die erhaltene Suspension mittels Druckfülltechnik in mit Dosier-Ventilen verschlossene Aluminiumdosen abgefüllt.

### Beispiel 15

1,6 g mikronisiertes Budesonid werden in einen Druckansatzkessel eingewogen. Nach Verschliessen und Evakuieren desselben wird eine Mischung aus 50 g Glycerin, 150 g Ethanol und 800 g HFA 134a zugegeben, die zuvor in einem anderen Druckansatzkessel mit Distickstoffoxid begast und auf einen Druck von 6,5 bar (20°C) eingestellt wurde. Nach Homogenisieren dieser Mischung wird die erhaltene Lösung mittels Druckfülltechnik in mit Dosier-Ventilen verschlossene Aluminiumdosen abgefüllt.

## Patentansprüche

1. Druckverflüssigtes Treibmittelgemisch für Aerosole, umfassend Distickstoffmonoxid und ein Hydrofluoralkan der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist, mit Ausnahme von Treibmittelgemischen, bestehend aus 1,1,1,2-Tetrafluorethan und einer im Vergleich zur Menge an 1,1,1,2-Tetrafluorethan kleineren Menge an Distickstoffmonoxid und/oder Kohlendioxid.

2. Treibmittelgemisch nach Anspruch 1, **dadurch gekennzeichnet**, es mindestens 40 Gew.-% an Hydrofluoralkan der Formel I enthält.

3. Treibmittelgemisch nach Anspruch 1 oder 2, **dadurch gekennzeichnet, dass** es mindestens 64 Gew.-%, vorzugsweise mindestens 87 Gew.-%, an Hydrofluoralkan der Formel I enthält.

4. Treibmittelgemisch nach einem der Ansprüche 1 bis 3, **dadurch gekennzeichnet, dass** es als Hydrofluoralkan der Formel I 1,1,1,2-Tetrafluorethan, 1,1,1,2,3,3,3-Heptafluorpropan oder ein Gemisch der beiden enthält.

5. Treibmittelgemisch nach einem der Ansprüche 1 bis 4, **dadurch gekennzeichnet, dass** es bei 20°C einen Druck von 3 bis 10 bar, vorzugsweise 3,5 bis 6 bar, aufweist.

6. Treibmittelgemisch nach einem der Ansprüche 1 bis 5, **dadurch gekennzeichnet, dass** es zusätzlich Kohlendioxid enthält.

7. Treibmittelgemisch nach einem der Ansprüche 1 bis 6, **dadurch gekennzeichnet, dass** der Gehalt an Distickstoffmonoxid mindestens 0,0001 Gew.-%, vorzugsweise mindestens 0,01 Gew.-%, beträgt.

8. Treibmittelgemisch nach einem der Ansprüche 1 bis 7, **dadurch gekennzeichnet, dass** der Gehalt an Distickstoffmonoxid oder der Gehalt an Distickstoffmonoxid und Kohlendioxid zusammen 0,0001 bis 10 Gew.-%, vorzugsweise 0,01 bis 6 Gew.-%, beträgt.

9. Treibmittelgemisch nach einem der Ansprüche 1 bis 8, **dadurch gekennzeichnet, dass** es zusätzlich ein Cosolvens in einer Menge von 0,01 bis 40 Gew.-%, vorzugsweise 0,1 bis 15 Gew.-%, enthält.

10. Treibmittelgemisch nach Anspruch 9, **dadurch gekennzeichnet, dass** es als Cosolvens Wasser, Ethanol, Propanol, Ethylenglykol, Propylenglykol, Glycerin, Propan, Butan, Isobutan, Pentan, Dimethylether oder Diethylether enthält.

11. Medizinische Aerosolformulierung, umfassend eine wirksame Menge eines pharmazeutischen Wirkstoffes und ein druckverflüssigtes Treibmittelgemisch, enthaltend Distickstoffmonoxid und ein Hydrofluoralkan der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist.

12. Aerosolformulierung nach Anspruch 11, **dadurch gekennzeichnet**, sie mindestens 30 Gew.-%, vorzugsweise mindestens 40 Gew.-%, an Hydrofluoralkan der Formel I enthält.

13. Aerosolformulierung nach Anspruch 11 oder 12, **dadurch gekennzeichnet, dass** sie mindestens 64 Gew.-%, vorzugsweise mindestens 87 Gew.-%, an Hydrofluoralkan der Formel I enthält.

14. Aerosolformulierung nach einem der Ansprüche 11 bis 13, **dadurch gekennzeichnet, dass** sie als Hydrofluoralkan der Formel I 1,1,1,2-Tetrafluorethan, 1,1,1,2,3,3,3-Heptafluorpropan oder ein Gemisch der beiden enthält.

15. Aerosolformulierung nach einem der Ansprüche 11 bis 14, **dadurch gekennzeichnet, dass** sie bei 20°C einen Druck von 3 bis 10 bar, vorzugsweise 3,5 bis 6 bar, aufweist.

16. Aerosolformulierung nach einem der Ansprüche 11 bis 15, **dadurch gekennzeichnet, dass** sie zusätzlich Kohlendioxid enthält.

17. Aerosolformulierung nach einem der Ansprüche 11 bis 16, **dadurch gekennzeichnet, dass** der Gehalt an Distickstoffmonoxid mindestens 0,0001 Gew.-%, vorzugsweise mindestens 0,01 Gew.-%, beträgt.

18. Aerosolformulierung nach einem der Ansprüche 11 bis 17, **dadurch gekennzeichnet, dass** der Gehalt an Distickstoffmonoxid oder der Gehalt an Distickstoffmonoxid und Kohlendioxid zusammen 0,0001 bis 10 Gew.-%, vorzugsweise 0,01 bis 6 Gew.-%, beträgt.

19. Aerosolformulierung nach einem der Ansprüche 11 bis 18, **dadurch gekennzeichnet, dass** sie zusätzlich ein Cosolvens in einer Menge von 0,01 bis 40 Gew.-%, vorzugsweise 0,1 bis 15 Gew.-%, enthält.

20. Aerosolformulierung nach Anspruch 19, **dadurch gekennzeichnet, dass** sie als Cosolvens Wasser, Ethanol, Propanol, Ethylenglykol, Propylenglykol, Glycerin, Propan, Butan, Isobutan, Pentan, Dimethylether und/oder Diethylether, vorzugsweise Ethanol und/oder Diethylether, enthält.

21. Aerosolformulierung nach einem der Ansprüche 11 bis 20, **dadurch gekennzeichnet, dass** sie ein oberflächenaktives Mittel, vorzugsweise Ölsäure, Lecithin, Sorbitantrioleat, Cetylpyridiniumchlorid, Benzalkoniumchlorid, Polyoxyethylen(20)sorbitanmonolaurat, Polyoxethylen (10) stearylether, Polyoxyethylen (2) oleylether, Polyoxyethylen (20) sorbitanmonostearat, Polyoxyethylen(20)sorbitanmonooleat, ein Polyoxypropylen-polyoxyethylen-Blockcopolymer, ein Polyoxypropylen-polyoxyethylen-ethylendiamin-Blockcopolymer oder ethoxyliertes Ricinusöl, enthält.

22. Aerosolformulierung nach einem der Ansprüche 11 bis 21, **dadurch gekennzeichnet, dass** sie 0,0001 bis 1 Gew.-%, vorzugsweise 0,001 bis 0,1 Gew.-%, an oberflächenaktiven Mitteln enthält.

23. Aerosolformulierung nach einem der Ansprüche 11 bis 21, **dadurch gekennzeichnet, dass** sie im wesentlichen frei von oberflächenaktiven Mitteln ist.

24. Aerosolformulierung nach einem der Ansprüche 11 bis 23, **dadurch gekennzeichnet, dass** sie als pharmazeutischen Wirkstoff Salbutamol, Formoterol, Salmeterol, Fluticason, Budesonid, Ciclesonid, Glycopyrronium, Tiotropium, Cromoglycinsäure, Nedocromil, Mometason, Sildenafil, Beclomethason, Levalbuterol oder ein pharmazeutisch annehmbares Salz oder Derivat davon enthalten.

25. Verfahren zur Herstellung einer medizinischen Aerosolformulierung gemäss Ansprüchen 11 bis 24, **dadurch gekennzeichnet, dass** man Distickstoffmonoxid unter Druck in ein verflüssigtes Hydrofluoralkan der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist, einleitet und den pharmazeutischen Wirkstoff zusetzt.

26. Verwendung eines druckverflüssigten Treibmittelgemisches gemäss Ansprüchen 1 bis 10 als Treibmittel für Aerosole.

27. Verwendung eines druckverflüssigten Treibmittelgemisches, umfassend Distickstoffmonoxid und ein Hydrofluoralkan der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist, für medizinische Aerosole, insbesondere für nasale und inhalative Aerosole.

28. Verwendung eines druckverflüssigten Treibmittelgemisches, umfassend Distickstoffmonoxid und ein Hydrofluoralkan der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist, in einem drucksicheren Behältnis mit einem Dosierventil und einem geeigneten Adapter zur vernebelung bzw. Inhalation von pharmazeutischen Wirkstoffen.

## Claims

1. A pressure-liquefied propellant mixture for aerosols, comprising dinitrogen monoxide and a hydrofluoroalkane of the general formula
CₓH_{y}F_{z} (I)
in which x is the number 1, 2 or 3, y and z are each an integer ≥ 1 and y + z = 2x + 2,
with the exception of propellant mixtures consisting of 1,1,1,2-tetrafluoroethane and a minor amount, in comparison to the amount of 1,1,1,2-tetrafluoroethane, of dinitrogen monoxide and/or carbon dioxide.

2. The propellant mixture according to claim 1, **characterized in that** it contains at least 40% by weight of hydrofluoroalkane of the formula I.

3. The propellant mixture according to claim 1 or 2, **characterized in that** it contains at least 64% by weight, preferably at least 87% by weight, of hydrofluoroalkane of the formula I.

4. The propellant mixture according to any one of claims 1 to 3, **characterized in that** it contains, as hydrofluoroalkane of the formula I, 1,1,1,2-tetrafluoroethane, 1,1,1,2,3,3,3-heptafluoropropane or a mixture of the two.

5. The propellant mixture according to any one of claims 1 to 4, **characterized in that** it has a pressure of 3 to 10 bar, preferably 3.5 to 6 bar, at 20°C.

6. The propellant mixture according to anyone of claims 1 to 5, **characterized in that** it additionally contains carbon dioxide.

7. The propellant mixture according to any one of claims 1 to 6, **characterized in that** the content of dinitrogen monoxide is at least 0.0001% by weight, preferably at least 0.01% by weight.

8. The propellant mixture according to any one of claims 1 to 7, **characterized in that** the content of dinitrogen monoxide or the content of dinitrogen monoxide and carbon dioxide together is 0.0001 to 10% by weight, preferably 0.01 to 6% by weight.

9. The propellant mixture according to any one of claims 1 to 8, **characterized in that** it additionally contains a cosolvent in an amount of from 0.01 to 40% by weight, preferably 0.1 to 15% by weight.

10. The propellant mixture according to claim 9, **characterized in that** it contains as cosolvent water, ethanol, propanol, ethylene glycol, propylene glycol, glycerol, propane, butane, isobutane, pentane, dimethyl ether or diethyl ether.

11. A medicinal aerosol formulation, comprising an efficacious amount of a pharmaceutically active compound and a pressure-liquefied propellant mixture, containing dinitrogen monoxide and a hydrofluoroalkane of the general formula
CₓH_{y}F_{z} (I)
in which x is the number 1, 2 or 3, y and z are each an integer ≥ 1 and y + z = 2x + 2.

12. The aerosol formulation according to claim 11, **characterized in that** it contains at least 30% by weight, preferably at least 40% by weight, of hydrofluoroalkane of the formula I.

13. The aerosol formulation according to claim 11 or 12, **characterized in that** it contains at least 64% by weight, preferably at least 87% by weight, of hydrofluoroalkane of the formula I.

14. The aerosol formulation according to any one of claims 11 to 13, **characterized in that** it contains, as hydrofluoroalkane of the formula I, 1,1,1,2-tetrafluoroethane, 1,1,1,2,3,3,3-heptafluoropropane or a mixture of the two.

15. The aerosol formulation according to any one of claims 11 to 14, **characterized in that** it has a pressure of 3 to 10 bar, preferably 3.5 to 6 bar, at 20°C.

16. The aerosol formulation according to any one of claims 11 to 15, **characterized in that** it additionally contains carbon dioxide.

17. The aerosol formulation according to any one of claims 11 to 16, **characterized in that** the content of dinitrogen monoxide is at least 0.0001% by weight, preferably at least 0.01% by weight.

18. The aerosol formulation according to any one of claims 11 to 17, **characterized in that** the content of dinitrogen monoxide or the content of dinitrogen monoxide and carbon dioxide together is 0.0001 to 10% by weight, preferably 0.01 to 6% by weight.

19. The aerosol formulation according to any one of claims 11 to 18, **characterized in that** it additionally contains a cosolvent in an amount of from 0.01 to 40% by weight, preferably 0.1 to 15% by weight.

20. The aerosol formulation according to claim 19, **characterized in that** it contains as cosolvent water, ethanol, propanol, ethylene glycol, propylene glycol, glycerol, propane, butane, isobutane, pentane, dimethyl ether and/or diethyl ether, preferably ethanol and/or diethyl ether.

21. The aerosol formulation according to any one of claims 11 to 20, **characterized in that** it contains a surface-active agent, preferably oleic acid, lecithin, sorbitan trioleate, cetylpyridinium chloride, benzalkonium chloride, polyoxyethylene (20) sorbitan monolaurate, polyoxyethylene (10) stearyl ether, polyoxyethylene (2) oleyl ether, polyoxyethylene (20) sorbitan monostearate, polyoxyethylene (20) sorbitan monooleate, a polyoxypropylene/polyoxyethylene block copolymer, a polyoxypropylene/polyoxyethylene/ethylenediamine block copolymer or ethoxylated castor oil.

22. The aerosol formulation according to any one of claims 11 to 21, **characterized in that** it contains 0.0001 to 1% by weight, preferably 0.001 to 0.1% by weight, of surface-active agents.

23. The aerosol formulation according to any one of claims 11 to 21, **characterized in that** it is essentially free of surface-active agents.

24. The aerosol formulation according to any one of claims 11 to 23, **characterized in that** it contains, as pharmaceutically active compound, salbutamol, formoterol, salmeterol, fluticasone, budesonide, ciclesonide, glycopyrronium, tiotropium, cromoglycic acid, nedocromil, mometasone, sildenafil, beclomethasone, levalbuterol or a pharmaceutically acceptable salt or derivative thereof.

25. A process for the preparation of a medicinal aerosol formulation as defined in claims 11 to 24, **characterized in that** dinitrogen monoxide is introduced under pressure into a liquefied hydrofluoroalkane of the general formula
CₓH_{y}F_{z} (I)
in which x is the number 1, 2 or 3, y and z are each an integer ≥ 1 and y + z = 2x + 2,
and the pharmaceutically active compound is added.

26. The use of a pressure-liquefied propellant mixture as defined in claims 1 to 10 as a propellant for aerosols.

27. The use of a pressure-liquefied propellant mixture, comprising dinitrogen monoxide and a hydrofluoroalkane of the general formula
CₓH_{y}F_{z} (I)
in which x is the number 1, 2 or 3, y and z are each an integer ≥ 1 and y + z = 2x + 2,
for medicinal aerosols, in particular for nasal and inhalant aerosols.

28. The use of a pressure-liquefied propellant mixture, comprising dinitrogen monoxide and a hydrofluoroalkane of the general formula
CₓH_{y}F_{z} (I)
in which x is the number 1, 2 or 3, y and z are each an integer ≥ 1 and y + z = 2x + 2,
in a pressure-resistant container having a metered-dose valve and a suitable adapter for the atomization or inhalation of pharmaceutically active compounds.

## Revendications

1. Mélange d'agents propulseurs liquéfiés sous pression pour aérosols, comprenant du protoxyde d'azote et un hydrofluoroalcane de formule générale
CₓH_{y}F_{z} (I)
où x représente le nombre 1, 2 ou 3, y et z signifient chacun un nombre entier ≥ 1 et y + z = 2x + 2, à l'exception de mélanges d'agents propulseurs constitués par le 1,1,1,2-tétrafluoroéthane et une quantité de protoxyde d'azote et/ou de dioxyde d'azote inférieure à la quantité de 1,1,1,2-tétrafluoroéthane.

2. Mélange d'agents propulseurs selon la revendication 1, **caractérisé en ce qu'**il contient au moins 40% en poids d'hydrofluoroalcane de formule I.

3. Mélange d'agents propulseurs selon la revendication 1 ou 2, **caractérisé en ce qu'**il contient au moins 64% en poids, de préférence au moins 87% en poids, d'hydrofluoroalcane de formule I.

4. Mélange d'agents propulseurs selon l'une quelconque des revendications 1 à 3, **caractérisé en ce qu'**il contient comme hydrofluoroalcane de formule I du 1,1,1,2-tétrafluoroéthane, du 1,1,1,2,3,3,3-heptafluoropropane ou un mélange des deux.

5. Mélange d'agents propulseurs selon l'une quelconque des revendications 1 à 4, **caractérisé en ce qu'**il présente à 20°C une pression de 3 à 10 bars, de préférence de 3,5 à 6 bars.

6. Mélange d'agents propulseurs selon l'une quelconque des revendications 1 à 5, **caractérisé en ce qu'**il contient en outre du dioxyde de carbone.

7. Mélange d'agents propulseurs selon l'une quelconque des revendications 1 à 6, **caractérisé en ce que** la teneur en protoxyde d'azote est d'au moins 0,0001% en poids, de préférence d'au moins 0,01% en poids.

8. Mélange d'agents propulseurs selon l'une quelconque des revendications 1 à 7, **caractérisé en ce que** la teneur en protoxyde d'azote et en dioxyde de carbone ensemble est de 0,0001 à 10% en poids, de préférence de 0,01 à 6% en poids.

9. Mélange d'agents propulseurs selon l'une quelconque des revendications 1 à 8, **caractérisé en ce qu'**il contient en outre un cosolvant en une quantité de 0,01 à 40% en poids, de préférence de 0,1 à 15% en poids.

10. Mélange d'agents propulseurs selon la revendication 9, **caractérisé en ce qu'**il contient comme cosolvant de l'eau, de l'éthanol, du propanol, de l'éthylèneglycol, du propylèneglycol, du glycérol, du propane, du butane, de l'isobutane, du pentane, du diméthyléther ou du diéthyléther.

11. Formulation médicale d'aérosol, comprenant une quantité active d'une substance active pharmaceutique et un mélange d'agents propulseurs liquéfié sous pression, contenant du protoxyde d'azote et un hydrofluoroalcane de formule générale
CₓH_{y}F_{z} (I)
où x représente le nombre 1, 2 ou 3, y et z signifient chacun un nombre entier ≥ 1 et y + z = 2x + 2.

12. Formulation d'aérosol selon la revendication 11, **caractérisée en ce qu'**elle contient au moins 30% en poids, de préférence au moins 40% en poids, d'hydrofluoroalcane de formule I.

13. Formulation d'aérosol selon la revendication 11 ou 12, **caractérisée en ce qu'**elle contient au moins 64% en poids, de préférence au moins 87% en poids, d'hydrofluoroalcane de formule I.

14. Formulation d'aérosol selon l'une quelconque des revendications 11 à 13, **caractérisée en ce qu'**elle contient comme hydrofluoroalcane de formule I du 1,1,1,2-tétrafluoroéthane, du 1,1,1,2,3,3,3-heptafluoropropane ou un mélange des deux.

15. Formulation d'aérosol selon l'une quelconque des revendications 11 à 14, **caractérisée en ce qu'**elle présente à 20°C une pression de 3 à 10 bars, de préférence de 3,5 à 6 bars.

16. Formulation d'aérosol selon l'une quelconque des revendications 11 à 15, **caractérisée en ce qu'**elle contient en outre du dioxyde de carbone.

17. Formulation d'aérosol selon l'une quelconque des revendications 11 à 16, **caractérisée en ce que** la teneur en protoxyde d'azote est d'au moins 0,0001% en poids, de préférence d'au moins 0,01% en poids.

18. Formulation d'aérosol selon l'une quelconque des revendications 11 à 17, **caractérisée en ce que** la teneur en protoxyde d'azote et en dioxyde de carbone ensemble est de 0,0001 à 10% en poids, de préférence de 0,01 à 6% en poids.

19. Formulation d'aérosol selon l'une quelconque des revendications 11 à 18, **caractérisée en ce qu'**elle contient en outre un cosolvant en une quantité de 0,01 à 40% en poids, de préférence de 0,1 à 15% en poids.

20. Formulation d'aérosol selon la revendication 19, **caractérisée en ce qu'**elle contient comme cosolvant de l'eau, de l'éthanol, du propanol, de l'éthylèneglycol, du propylèneglycol, du glycérol, du propane, du butane, de l'isobutane, du pentane, du diméthyléther ou/ou du diéthyléther, de préférence de l'éthanol et/ou du diéthyléther.

21. Formulation d'aérosol selon l'une quelconque des revendications 11 à 20, **caractérisée en ce qu'**elle contient un agent tensioactif, de préférence l'acide oléique, la lécithine, le trioléate de sorbitane, le chlorure de cétylpyridinium, le chlorure de benzalconium, le monolaurate de polyoxyéthylène(20)sorbitane, le polyoxyéthylène(10)stéaryléther, le polyoxyéthylène(2)oléyléther, le monostéarate de polyoxyéthylène(20)sorbitane, le monooléate de polyoxyéthylène(20)sorbitane, un copolymère à blocs de polyoxypropylène-polyoxyéthylène, un copolymère à blocs de polyoxypropylènepolyoxyéthylène-éthylènediamine ou l'huile de ricin éthoxylée.

22. Formulation d'aérosol selon l'une quelconque des revendications 11 à 21, **caractérisée en ce qu'**elle contient 0,0001 à 1% en poids, de préférence 0,001 à 0,1% en poids, d'agents tensioactifs.

23. Formulation d'aérosol selon l'une quelconque des revendications 11 à 21, **caractérisée en ce qu'**elle est essentiellement exempte d'agents tensioactifs.

24. Formulation d'aérosol selon l'une quelconque des revendications 11 à 23, **caractérisée en ce que** qu'elle contient comme substance active pharmaceutique le salbutamol, le formotérol, le salmétérol, le fluticasone, le budésonide, le ciclésonide, le glycopyrronium, le tiotropium, l'acide cromoglycinique, le nédocromil, le mométasone, le sildénafil, le béclomethasone, le lévalbutérol ou un sel ou un dérivé pharmaceutiquement acceptable de ceux-ci.

25. Procédé pour la préparation d'une formulation médicale d'aérosol selon les revendications 11 à 24, **caractérisé en ce qu'**on introduit du protoxyde d'azote sous pression dans un hydrofluoroalcane liquéfié de formule générale
CₓH_{y}F_{z} (I)
où x représente le nombre 1, 2 ou 3, y et z signifient chacun un nombre entier ≥ 1 et y + z = 2x + 2 et on ajoute la substance active pharmaceutique.

26. Utilisation d'un mélange d'agents propulseurs liquéfiés sous pression selon les revendications 1 à 10 comme agent propulseur pour des aérosols.

27. Utilisation d'un mélange d'agents propulseurs liquéfié sous pression, comprenant du protoxyde d'azote et un hydrofluoroalcane de formule générale
CₓH_{y}F_{z} (I)
où x représente le nombre 1, 2 ou 3, y et z signifient chacun un nombre entier ≥ 1 et y + z = 2x + 2, pour des aérosols médicaux, en particulier pour des aérosols nasaux et d'inhalation.

28. Utilisation d'un mélange d'agents propulseurs liquéfiés sous pression, comprenant du protoxyde d'azote et un hydrofluoroalcane de formule générale
CₓH_{y}F_{z} (I)
où x représente le nombre 1, 2 ou 3, y et z signifient chacun un nombre entier ≥ 1 et y + z = 2x + 2, dans un récipient résistant à la pression avec une soupape de dosage et un adaptateur approprié pour la nébulisation ou l'inhalation de substances actives pharmaceutiques.
